# EUROPEAN PATENT APPLICATION

(11) **EP 4 495 218 A1**
(43) Date of publication of application: **22.01.2025**
(21) Application number: 22937097.8
(22) Date of filing: 29.06.2022
(51) Int. Cl.: C12M 1/00, C12M 1/24, C12M 1/34

(54) **REAGENT CARTRIDGE, DETECTION DEVICE, AND DETECTION METHOD**

(30) Priority: 15.04.2022 CN 202210399871
(71) Applicant: GUANGDONG RUNPENG BIOLOGICAL TECHNOLOGY CO., LTD., Dongguan, Guangdong 523000 (CN)
(72) Inventor: HUANG, Chaojie, Dongguan, Guangdong 523000 (CN); HUANG, Hongkun, Dongguan, Guangdong 523000 (CN)
(74) Representative: HGF
(86) International application number: PCT/CN2022/102410
(87) International publication number: WO 2023/197459

(57) **Abstract**

Provided are a reagent cartridge, a detection device, and a detection method. The reagent cartridge includes: a cartridge body assembly, a plurality of accommodating chambers are formed in the cartridge body assembly, a sealing layer is arranged at a chamber opening of at least one of the plurality of accommodating chambers, the plurality of accommodating chambers include an eluent chamber, and a communication part is formed at the bottom of the eluent chamber; a tube member, the tube member is connected to the cartridge body assembly; a first flow channel, the first flow channel is in conductive communication from the communication part to the tube member; and an on-off assembly, the on-off assembly is arranged on the first flow channel, when the on-off assembly is located at a first position, the first flow channel is in conductive communication, and when the on-off assembly is located at a second position, the first flow channel is closed.

## Description

### Cross-Reference to Related Application

The present invention claims priority to Chinese Patent Application No. 202210399871.X, filed to the China National Intellectual Property Administration on April, 15, 2022 and entitled "Reagent Cartridge, Detection Device, and Detection Method", the present invention of which is hereby incorporated by reference in its entirety.

### Technical Field

The present invention relates to the technical field of nucleic acid detection, and in particular, to a reagent cartridge, a detection device, and a detection method.

### Background

Since the outbreak of the novel coronavirus, the demand for molecular detection technologies, led by a fluorescence Quantitative Polymerase Chain Reaction (qPCR) technology, has seen a spurt in growth. Traditional molecular detection steps mainly include nucleic acid extraction, reagent packaging, qPCR instrument testing, and result analysis.

However, these steps first need to complete the above detection process in a professional physically partitioned qPCR molecular laboratory. Secondly, the laboratory needs to be equipped with expensive instruments including a nucleic acid extractor, a biosafety cabinet, a fluorescent qPCR instrument, etc. Then, it is necessary to rely on specially trained professional operators to complete the detection.

In general, traditional molecular detection reagents have problems such as expensive investment in the construction and operation of the detection platform, expensive device, the need for professional operators, cumbersome manual operation, long detection cycle, easy pollution, and risks of harm to the safety of operators. These problems have resulted in only some tertiary hospitals or large hospitals being qualified, qualified, and capable of carrying out related molecular detection projects, while many secondary hospitals and primary medical institutions are unable to carry out related detection projects. After the outbreak of the novel coronavirus, detection of primary hospitals and on-site detection are the key to preventing the spread of infectious diseases.

Currently, there are various molecular Point Of Care Testing (POCT) products developed based on a microfluidic chip technology on the market. Most of these products achieve the transfer of a liquid between different chambers by creating negative pressure or using centrifugation. In an embodiment, Cepheid's GeneXpert products and BioMerieux's filmArray drive the liquid to transfer and flow by pushing and pulling a pump body to change the air pressure. These classic microfluidic designs have solved the impact of liquid residue on subsequent amplification steps through subsequent reagent optimization. These manners that rely on the microfluidic design to drive the liquid transfer have imposed restrictions on the volume of manipulated liquids, especially on the sample volume, which results in low detection sensitivity and poor stability of the product, greatly reducing the application value of the final product.

However, due to the complex structure of POCT cartridges at this stage, the cost increases, and the detection process is cumbersome, resulting in reduced detection efficiency.

Therefore, there is an urgent need to provide a reagent cartridge, a detection device, and a detection method to solve the problems existing in the related art to a certain extent.

### Summary

Some embodiments of the present invention provide a reagent cartridge, a detection device, and a detection method, so as to at least solve the problem that pollution is easily generated in a nucleic acid detection process at the preset stage.

A reagent cartridge provided by some embodiments of the present invention include a cartridge body assembly, a tube member, a first flow channel, and an on-off assembly. A plurality of accommodating chambers may be formed in the cartridge body assembly, a sealing layer may be arranged at a chamber opening of at least one of the plurality of accommodating chambers, the plurality of accommodating chambers include an eluent chamber, and a communication part may be formed at the bottom of the eluent chamber. The tube member may be connected to the cartridge body assembly. The first flow channel may be in conductive communication from the communication part to the tube member. The on-off assembly may be arranged on the first flow channel, when the on-off assembly is located at a first position, the first flow channel is in conductive communication, and when the on-off assembly is located at a second position, the first flow channel is closed.

The cartridge body assembly include a cartridge body and a connection module. The plurality of accommodating chambers may be formed in the cartridge body, and the connection module may be connected to one end, close to the eluent chamber, of the cartridge body. The first flow channel may be formed in the connection module, a second flow channel may further be formed in the connection module, one end of the second flow channel may communicate with the tube member, a piston chamber may be formed in the connection module, one end, away from the tube member, of the second flow channel may communicate with the piston chamber, and a movable member that is able to move relative to the piston chamber may be arranged in the piston chamber.

In an embodiment, a diameter of the first flow channel may be 1 mm to 2 mm.

In an embodiment, the reagent cartridge provided by the present invention further include a magnetic rod sleeve assembly. The magnetic rod sleeve assembly may be arranged in one of the plurality of accommodating chambers.

The plurality of accommodating chambers may be a magnetic rod sleeve chamber, sample chambers, a magnetic bead preservation liquid chamber, and a washing liquid chamber group. The magnetic rod sleeve chamber, the sample chamber, the magnetic bead preservation liquid chamber, the washing liquid chamber group, and the eluent chamber may be sequentially arranged from one end, away from the connection module, of the cartridge body to one end, close to the connection module, of the cartridge body.

In an embodiment, the washing liquid chamber group may at least include a first washing liquid chamber, a second washing liquid chamber, and a standby chamber. The first washing liquid chamber may be arranged close to the magnetic bead preservation liquid chamber, and the second washing liquid chamber and the standby chamber may be sequentially arranged in a direction away from the first washing liquid chamber.

In an embodiment, at least two parallel magnetic rod sleeve chambers, at least two parallel sample chambers, at least two parallel magnetic bead preservation liquid chambers, at least two rows of parallel washing liquid chamber groups, and at least two parallel eluent chambers may be formed in the cartridge body.

At least two first flow channels, at least two piston chambers, and at least two second flow channels may be formed in the connection module, and the at least two tube members are connected to the connection module.

In an embodiment, the on-off assembly may be movably connected to the connection module. The on-off assembly may separate the first flow channel into a first circulation section and a second circulation section, and an alignment hole may be formed in the on-off assembly. When the on-off assembly moves relative to the connection module to align the alignment hole with a port of the first circulation section and a port of the second circulation section, the first circulation section is conductive communication with the second circulation section.

In an embodiment, a positioning part is formed at a position of the connection module corresponding to the tube member, and a tube opening of the tube member may be sleeved on the positioning part.

A first flow guide tube and a second flow guide tube may be formed on the positioning part, the first flow channel may be formed in the first flow guide tube, and the second flow channel may be formed in the second flow guide tube.

The reagent cartridge provided by the present invention further include a slide cover. The slide cover may be slidably connected to the cartridge body, and the chamber openings of the plurality of accommodating chambers may be covered by the slide cover in a length direction of the cartridge body. In an embodiment, an alignment part, a first perforation, and a second perforation may be formed in the slide cover, the alignment part, the first perforation, and the second perforation may be sequentially arranged from one end of the slide cover to the other end in a length direction of the slide cover, and a distance from the second perforation to one end, away from the second perforation, of the slide cover is not less than a distance from the sample chamber to one end, away from the sample chamber, of the cartridge body.

In an embodiment, a first matching part may be formed on the cartridge body, and the first matching part may extend in the length direction of the cartridge body. A second matching part is formed at a position of the slide cover corresponding to the first matching part, and the first matching part may be matched with the second matching part, so that the slide cover may slide relative to the cartridge body.

A matching protrusion may be formed at one end of the cartridge body, and a matching recess may be formed at a position of the connection module corresponding to the matching protrusion. A third matching part and a fourth matching part may be respectively formed on two side walls, in contact with the matching recess, of the matching protrusion, a fifth matching part matched with the third matching part may be formed at a position of the matching recess corresponding to the third matching part, and a sixth matching part matched with the fourth matching part may be formed at a position corresponding to the fourth matching part.

In an embodiment, the third matching part and the fourth matching part may both be matching buckles, and the fifth matching part and the sixth matching part may both be matching slots.

In an embodiment, a first sealing gasket may be arranged between the matching protrusion and the matching recess, the eluent chamber may be formed in the matching protrusion, and one end of the first flow channel may pass through the matching recess. A conductive communication part may be formed at a position of the first sealing gasket corresponding to the communication part, and one end of the conductive communication part communicates with the communication part while the other end thereof communicates with the first flow channel.

In an embodiment, the magnetic rod sleeve assembly include a sleeve body, a fixed section, a side wing, and an anti-lost magnetic ring. The fixed section may be connected to one end of the sleeve body, a through hole may be formed in the fixed section, an accommodating groove may be formed in the sleeve body, the through hole may communicate with the accommodating groove, and a puncture tip may be formed at the other end of the sleeve body. The side wing may extend in a length direction of the sleeve body and be arranged on an outer wall surface of the sleeve body. The anti-lost magnetic ring may be arranged at one end, away from the fixed section, of the sleeve body and form an accommodating recess with the puncture tip.

The movable member include a fixed part, a movable part, and a ring sleeve part. The fixed part may be rotatably connected to an open end of the piston chamber, one end of the movable part may be rotatably connected to the fixed part, the ring sleeve part may be sleeved on the other end of the movable part, and an outer wall of the ring sleeve part may abut against an inner wall of the piston chamber. When the fixed part rotates relative to the piston chamber, the movable part may move in an axial direction of the piston chamber to drive the ring sleeve part to move relative to the piston chamber.

Compared with the related art, the reagent cartridge provided by the present invention has at least the following advantages.

The reagent cartridge provided by the present invention includes the cartridge body assembly, the tube member, the first flow channel, and the on-off assembly. The plurality of accommodating chambers are formed in the cartridge body assembly, the sealing layer is arranged at the chamber opening of at least one of the plurality of accommodating chambers, the plurality of accommodating chambers include the eluent chamber, and the communication part is formed at the bottom of the eluent chamber. The tube member is connected to the cartridge body assembly. The first flow channel is in conductive communication from the communication part to the tube member. The on-off assembly is arranged on the first flow channel, when the on-off assembly is located at the first position, the first flow channel is in conductive communication, and when the on-off assembly is located at the second position, the first flow channel is closed.

From this analysis, it is able to be seen that, the plurality of accommodating chambers formed in the cartridge body may carry a sample to be detected, a lysis binding solution, a magnetic bead preservation liquid, a washing liquid, an eluent, and other solvents to be applied in nucleic acid detection. A sealing film covering the chamber openings of the accommodating chambers may seal and protect the various solvents in the accommodating chambers, and since the reagent cartridge provided by the present invention is a disposable product, when the corresponding accommodating chamber needs to be used, the sealing film may be punctured for use.

The reagent cartridge in the present invention further includes the on-off assembly located between the cartridge body assembly and the tube member, so that the on-off assembly may perform on-off control on the first flow channel. When a reagent in the eluent chamber needs to enter the tube member, the on-off assembly moves to the first position, so that the first flow channel is in conductive communication, and the reagent in the eluent chamber enters the tube member. When the reagent in the eluent chamber needs to be blocked from entering the tube member, the on-off assembly moves to the second position, so that the first flow channel is disconnected to block the reagent from entering the tube member.

The reagent cartridge provided by the present invention is simple in structure and easy to operate, and may quickly complete the sample detection. Accordingly, the detection efficiency may be improved and the manufacturing cost of the overall structure may be reduced.

Some other embodiments of the present invention further provide a detection device. The detection device include a detection module, a pipette, and the reagent cartridge of the above embodiments. The pipette injects a sample solvent to be detected into one of the plurality of accommodating chambers of the reagent cartridge, the reagent cartridge is connected to the detection module, and the sample solvent to be detected in the one of plurality of accommodating chambers may be detected through the detection module.

When the detection device provided by the present invention is in use, the sample solvent to be detected is injected into the sample chamber of the reagent cartridge through the pipette, and then the reagent cartridge carrying the sample solvent to be detected is installed on the detection module, so that the sample solvent to be detected may be detected through the detection module.

The detection module provided by the present invention include a detection module body, a magnetic rod, a moving mechanism, a first pushing member, a second pushing member, a rotating assembly, and other structures. The detection module body may carry the reagent cartridge, the magnetic rod may be inserted into a magnetic rod sleeve assembly of the reagent cartridge to adsorb magnetic beads carried in one of the plurality of accommodating chambers in a cartridge body of the reagent cartridge, the moving mechanism may grab and move the magnetic rod sleeve assembly, the first pushing member may push a slide cover of the reagent cartridge to slide relative to the cartridge body, and the second pushing member may push an on-off assembly of the reagent cartridge in a connection module of the reagent cartridge to move relative to the connection module to achieve the on-off of a first flow channel of the reagent cartridge, so that a sample may be detected quickly and safely through the structures contained in the above detection module.

Some further embodiments of the present invention further provide a detection method. The detection method include the following steps that: a detection device receives a reagent cartridge, a sample is injected into a sample chamber of the reagent cartridge, and a sealing layer is arranged at a chamber opening of the sample chamber; the detection device removes a magnetic rod sleeve assembly from the reagent cartridge, extends a magnetic rod of the detection device into the magnetic rod sleeve assembly and then sequentially enters a magnetic bead preservation liquid chamber, a sample chamber, a washing liquid chamber group, and an eluent chamber of the reagent cartridge; the detection device pushes an on-off assembly of the reagent cartridge to be in conductive communication with a first flow channel, and the first flow channel communicates with the eluent chamber and a tube member; the detection device drives a piston assembly communicating with the tube member on the reagent cartridge to form a negative pressure in the tube member, so that a solvent in the eluent chamber enters the tube member through the first flow channel to redissolve a freeze-dried amplification reagent in the tube member; and the detection device performs detection and analysis on the reagent in the tube member.

In an embodiment, the detection method provided by the above embodiments of the present invention further include that: the detection device controls the movement of a slide cover slidably connected to a cartridge body of the reagent cartridge to sequentially seal the magnetic bead preservation liquid chamber, the sample chamber, the washing liquid chamber group, and the eluent chamber after the magnetic rod sleeve assembly leaves.

Through the above method, rapid detection of the sample may be achieved, and due to the movement of a sealing film and the slide cover before and during the detection, the sample and the solvent in each accommodating chamber may be kept in a sealed state, so that the problem that the detection environment is affected due to aerosol transmission of the solvent in the accommodating chamber after the sealing film is destroyed may be avoided, thereby improving the detection accuracy.

### Brief Description of the Drawings

In order to more clearly illustrate the specific implementations of the present invention or the technical solutions in the related art, the drawings used in the description of the specific implementations or the related art will be briefly described below. It is apparent that the drawings described below are only some implementations of the present invention. Other drawings may further be obtained by those of ordinary skill in the art according to these drawings without creative efforts.
Fig. 1 is a schematic diagram of an overall structure of a reagent cartridge provided by an embodiment of the present invention.
Fig. 2 is an exploded schematic diagram of a reagent cartridge provided by an embodiment of the present invention.
Fig. 3 is a section view of a reagent cartridge provided by an embodiment of the present invention.
Fig. 4 is a schematic structural diagram of a cartridge body in a reagent cartridge provided by an embodiment of the present invention from a first perspective.
Fig. 5 is a schematic structural diagram of a cartridge body in a reagent cartridge provided by an embodiment of the present invention from a second perspective.
Fig. 6 is a schematic structural diagram of a slide cover in a reagent cartridge provided by an embodiment of the present invention from a first perspective.
Fig. 7 is a schematic structural diagram of a slide cover in a reagent cartridge provided by an embodiment of the present invention from a third perspective.
Fig. 8 is a schematic structural diagram of a magnetic rod sleeve assembly in a reagent cartridge provided by an embodiment of the present invention.
Fig. 9 is a schematic structural diagram of a magnetic rod sleeve assembly in a reagent cartridge provided by an embodiment of the present invention from a second perspective.
Fig. 10 is a schematic structural diagram of a front end of a magnetic rod sleeve assembly in a reagent cartridge provided by an embodiment of the present invention.
Fig. 11 is a schematic structural diagram of a connection module in a reagent cartridge provided by an embodiment of the present invention.
Fig. 12 is a schematic diagram of an internal structure of a connection module in a reagent cartridge provided by an embodiment of the present invention.
Fig. 13 is a section view of a connection module in a reagent cartridge provided by an embodiment of the present invention.
Fig. 14 is a schematic structural diagram of a movable member in a reagent cartridge provided by an embodiment of the present invention.
Fig. 15 is a section view of a movable member in a reagent cartridge provided by an embodiment of the present invention.
Fig. 16 is a flowchart of a detection method provided by an embodiment of the present invention.
Fig. 17 is an amplification cycle diagram provided by an embodiment of the present invention.

In the figures: 1-Cartridge body; 101-Magnetic rod sleeve chamber; 102-Sample chamber; 103-Magnetic bead preservation liquid chamber; 104-First washing liquid chamber; 105-Second washing liquid chamber; 106-Standby chamber; 107-Eluent chamber; 108-First matching part; 109-Matching protrusion; 1091-Third matching part; 1092-Fourth matching part; 1093-Communication part; 2-Slide cover; 201-Alignment hole; 202-First perforation; 203-Second perforation; 204-Second matching part; 3-Magnetic rod sleeve assembly; 301-Fixed section; 302-Sleeve body; 303-Side wing; 304-Puncture tip; 305-Anti-lost magnetic ring; 306-Accommodating recess; 4-Connection module; 401-First flow channel; 4011-First circulation section; 4012-Second circulation section; 402-On-off assembly; 403-Second flow channel; 404-Piston chamber; 405-Matching recess; 4051-Fifth matching part; 4052-Sixth matching part; 406-Positioning part; 4061-First flow guide tube; 4062-Second flow guide tube; 407-Forming hole; 4071-Blocking member; 5-Movable member; 501-Fixed part; 502-Movable part; 5021-Bearing groove; 503-Ring sleeve part; 6-Amplification tube; 7-First sealing gasket; 8-Second sealing gasket.

### Detailed Description of the Embodiments

The technical solutions of the present invention will be clearly and completely described in conjunction with the drawings. It is apparent that the described embodiments are only a part of the embodiments of the present invention, and not all of them. All other embodiments obtained by those of ordinary skill in the art on the basis of the embodiments in the present invention without creative work shall fall within the scope of protection of the present invention.

In the description of the present invention, it is to be noted that the orientations or positional relationships indicated by the terms "upper", "down", "left", "right", "vertical", "horizontal", "inside", "outside", etc. are based on the orientations or positional relationships shown in the drawings, and are only for the convenience of describing the present invention and simplifying the description. The description does not indicate or imply that the system or element referred to must have a specific orientation, be constructed and operated in a specific orientation, and therefore cannot be construed as limiting the present invention. In addition, terms "first" and "second" are only configured for describing purposes, and cannot be understood as indicating or implying relative importance.

In the description of the present invention, it is to be noted that, unless otherwise clearly specified and limited, the terms "installation", "mutual connection", "connection", and other terms shall be understood in a broad sense. In an embodiment, the term may be a fixed connection or a detachable connection, or an integrated connection; the term may be a mechanical connection or an electric connection; and the term may be a direct connection or an indirect connection through an intermediary, and may be communication inside two components. The specific meaning of the above-mentioned terminology in the present invention may be understood by those of ordinary skill in the art in specific circumstances.

### Embodiment 1

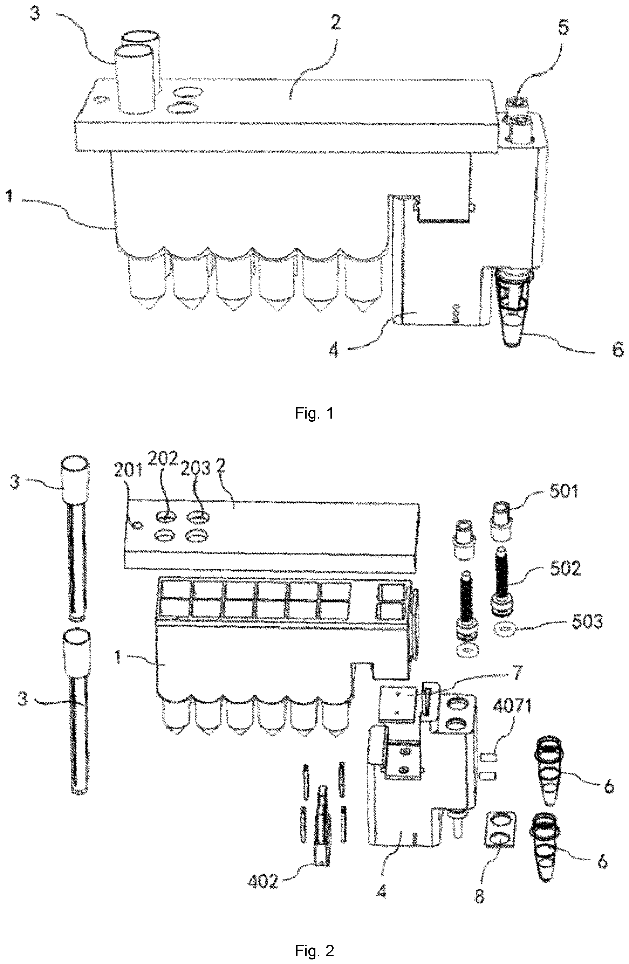

As shown in Fig. 1, the present invention provides a reagent cartridge, including a cartridge assembly and a tube member. The cartridge assembly includes a cartridge body 1 and a connection module 4, and the tube member in the present invention is an amplification tube 6. The connection module 4 is connected to one end of the cartridge body 1, and the amplification tube 6 is connected to the connection module 4. A plurality of accommodating chambers are formed in the cartridge body 1, a sealing layer is arranged at chamber openings of the plurality of accommodating chambers, the sealing layer is an aluminum sealing film covering the chamber openings of the plurality of accommodating chambers in a hot melting manner, the accommodating chamber close to the connection module 4 is an eluent chamber 107, a communication part 1093 is formed at the bottom of the eluent chamber 107, a first flow channel 401 is formed at a position of the connection module 4 corresponding to the communication part 1093, and one end of the first flow channel 401 is in conductive communication with the communication part 1093 while the other end thereof extends into the amplification tube 6. A second flow channel 403 and a piston chamber 404 are formed in the connection module 4, a movable member 5 is arranged in the piston chamber 404, and one end of the second flow channel 403 communicates with the piston chamber 404 while the other end thereof communicates with the amplification tube 6.

Compared with the related art, the reagent cartridge provided by the present invention has at least the following advantages.

According to the reagent cartridge provided by the present invention, the plurality of accommodating chambers formed in the cartridge body 1 may carry a sample to be detected, a lysis binding solution, a magnetic bead preservation liquid, a washing liquid, an eluent, and other solvents to be applied in nucleic acid detection. The sealing film covering the chamber openings of the plurality of accommodating chambers may seal and protect the various solvents in the plurality of accommodating chambers, and since the reagent cartridge provided by the present invention is a disposable product, when the corresponding accommodating chamber needs to be used, the sealing film may be punctured for use. Therefore, in this implementation, it may only be ensured that the solvent in the accommodating chamber is not polluted by the external environment before the sealing film is punctured, and no aerosol transmission occurs.

The reagent cartridge in the present invention further includes the connection module 4 connected to the cartridge body 1. The first flow channel 401 communicating with the eluent chamber 107 is formed in the connection module 4, the other end of the first flow channel 401 extends into the amplification tube 6, and the second flow channel 403 extending into the amplification tube 6 and the piston chamber 404 communicating with the second flow channel 403 are further formed in the connection module 4, so that gas in the amplification tube 6 may be extracted through the movement of the movable member 5 relative to the piston chamber 404, a negative pressure is generated in the amplification tube 6, and the solvent in the eluent chamber 107 is drawn into the amplification tube 6 through the first flow channel 401.

Before and during the detection of the reagent cartridge provided by the present invention, when the sealing film is not punctured, unused solvents and uncollected samples in the plurality of accommodating chambers are not in contact with the external environment, so that the pollution from the external environment may be avoided to a certain extent, thereby improving the detection accuracy.

It is to be noted here that the PCR amplification tube 6 in the present invention may be a conventional PCR tube or a customized PCR tube. The amplification tube 6 is filled with an amplification reagent, and the nucleic acid amplification detection reagent includes components such as reverse transcriptase, hot start Taq enzyme, dNTPs, primers and probes, which are directly formed at the bottom of the PCR amplification tube 6 by freeze drying. The reagents contained in the plurality of accommodating chambers of the cartridge body 1 mainly include a lysis binding solution, a first washing liquid, a second washing liquid, magnetic beads, an eluent, a proteinase K, etc. Therefore, accordingly, the plurality of accommodating chambers in the present invention are a magnetic rod sleeve chamber 101, a sample chamber 102, a magnetic bead preservation liquid chamber 103, a first washing liquid chamber 104, a second washing liquid chamber 105, and a standby chamber 106, and the magnetic rod sleeve chamber 101, the sample chamber 102, the magnetic bead preservation liquid chamber 103, the first washing liquid chamber 104, the second washing liquid chamber 105, the standby chamber 106, and the eluent chamber 107 are sequentially arranged from one end, away from the connection module 4, of the cartridge body 1 to the other end.

The magnetic rod sleeve chamber 101 is configured to carry the magnetic rod sleeve assembly 3, the sample chamber 102 is configured to contain a sample solvent, the magnetic bead preservation liquid chamber 103 is configured to contain a magnetic bead preservation liquid, and the magnetic bead preservation liquid contains the magnetic beads. The first washing liquid chamber 104 and the second washing liquid chamber 105 are configured to accommodate the first washing liquid and the second washing liquid respectively, the standby chamber 106 is also configured to contain the washing liquid, and the eluent chamber 107 is configured to contain the eluent.

Before detection, the sample solvent is first injected into the sample chamber 102, and then film sealing is performed on the chamber opening of the sample chamber 102 again. Then, the reagent cartridge is installed in a detection module for detection.

During detection, the detection module pulls the magnetic rod sleeve assembly 3 out of the magnetic rod sleeve chamber 101 and moves the magnetic rod sleeve assembly 3 to above the magnetic bead preservation liquid chamber 103. After a magnetic rod of the detection module is inserted into the magnetic rod sleeve assembly 3, the magnetic rod sleeve assembly 3 penetrates the sealing film of the magnetic bead preservation liquid chamber 103 and enters the chamber to adsorb the magnetic beads.

In this implementation, a washing liquid chamber group includes the first washing liquid chamber 104, the second washing liquid chamber 105, and the standby chamber 106. Therefore, after the magnetic beads in the magnetic bead preservation liquid chamber 103 are absorbed, the magnetic rod sleeve assembly 3 sequentially enters the sample chamber 102, the first washing liquid chamber 104, the second washing liquid chamber 105, and the standby chamber 106 from the magnetic bead preservation liquid chamber 103, and finally enters the eluent chamber 107.

Since the magnetic rod sleeve chamber 101, the sample chamber 102, the magnetic bead preservation liquid chamber 103, the first washing liquid chamber 104, the second washing liquid chamber 105, the standby chamber 106, and the eluent chamber 107 are sequentially arranged from one end, away from the connection module 4, of the cartridge body 1 to the other end, the detection operation of the detection module may be facilitated to a certain extent, thereby improving the operation efficiency.

### Embodiment 2

In the above implementation, the plurality of accommodating chambers may be sequentially arranged in a length direction of the cartridge body 1 to form a structure of the cartridge body 1 with a single row of plurality of accommodating chambers, so that only a single sample may be detected. However, due to the lack of positive and negative controls in the process of detecting the single sample, especially negative control, once aerosol pollution occurs in the detection environment, it is difficult to determine whether some weak positive results are false positives caused by pollution or true weak positives due to the lack of negative control, thereby greatly reducing the authority of the diagnosis results.

Therefore, based on the above implementation, the present invention further optimizes the structure of the cartridge body 1 and provides a second implementation.

As shown in Figs. 1 -5, at least two magnetic rod sleeve chambers 101, at least two sample chambers 102, at least two magnetic bead preservation liquid chambers 103, at least two first washing liquid chambers 104, at least two second washing liquid chambers 105, at least two standby chambers 106, and at least two eluent chambers 107 are formed in the cartridge body 1.

Optionally, two parallel magnetic rod sleeve chambers 101, two parallel sample chambers 102, two parallel magnetic bead preservation liquid chambers 103, and two rows of parallel washing liquid chamber groups are formed in the cartridge body 1 in the present invention, so as to have two parallel first washing liquid chambers 104, two parallel second washing liquid chambers 105, two parallel standby chambers 106, and two parallel eluent chambers 107, that is, the cartridge body 1 provided by the present invention has double rows of accommodating chambers, the reagents contained in the two rows of accommodating chambers are the same and have synchronous operation actions, one row is configured for sample detection, and the other row is configured as negative control reagents, so that each sample detection has a real negative control as a reference, thereby guaranteeing the reliability of the results.

In an embodiment, in the present invention, at least two first flow channels 401, at least two piston chambers 404, and at least two second flow channels 403 are formed in the connection module 4, and at least two amplification tubes 6 are connected to the connection module 4. One end of the first flow channel 401 is in conductive communication with the eluent chamber 107 correspondingly while the other end thereof is in conductive communication with the amplification tube 6 correspondingly, and one end of the second flow channel 403 is in conductive communication with the piston chamber 404 correspondingly while the other end thereof is in conductive communication with the amplification tube 6 correspondingly.

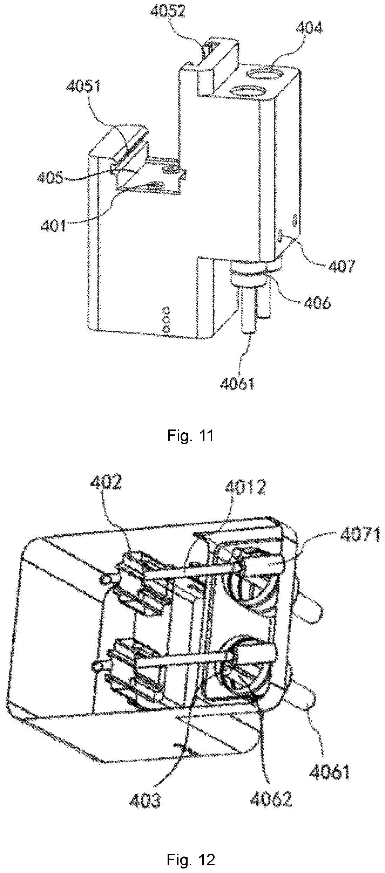

In the present invention, the first flow channel 401, the second flow channel 403, and the piston chamber 404 formed in the connection module 4 all correspond to the number of rows of the above plurality of accommodating chambers, and therefore, as shown in Fig. 11, two first flow channels 401, two second flow channels 403, and two piston chambers 404 are formed in the connection module 4 in the present invention. Accordingly, two amplification tubes 6 are correspondingly connected to the connection module 4, so that the synchronous actions of sample detection and negative control may be achieved.

In an embodiment, a diameter of the first flow channel 401 in the present invention is 1 mm to 2 mm. Since the diameter of the first flow channel 401 is relatively small, under the action of atmospheric pressure, the solvent in the eluent chamber 107 may be prevented from entering the first flow channel 401 in advance during mixing to affect the mixing effect to a certain extent. When the movable member 5 moves in the piston chamber 404, a negative pressure may be formed in the amplification tube 6, so that the mixed solvent may flow into the amplification tube 6 through the first flow channel 401.

It is to be noted here that the washing liquid chamber group in the present invention includes the first washing liquid chamber, the second washing liquid chamber, and the standby chamber, which is only one of the implementations required based on the detected sample. The washing liquid chamber group may further be provided with only the first washing liquid chamber, or the first washing liquid chamber and the second washing liquid chamber, or more washing liquid chambers and standby chambers. The specific number of washing liquid chambers and standby chambers is determined by the characteristics of the detected sample.

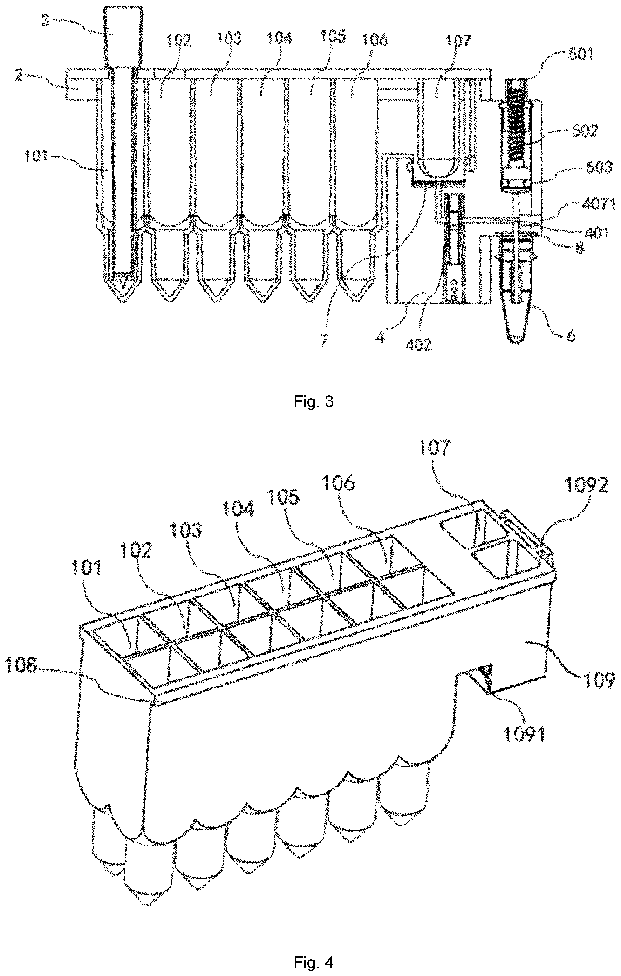

In an embodiment, as shown in Fig. 3 in combination with Figs. 12 -13, in the present invention, the on-off assembly 402 is arranged in the connection module 4, and the on-off assembly 402 is movably connected to the connection module 4. The on-off assembly 402 separates the first flow channel 401 into a first circulation section 4011 and a second circulation section 4012, and an alignment hole is formed in the on-off assembly 402. When the on-off assembly 402 moves relative to the connection module 4 to align the alignment hole with a port of the first circulation section 4011 and a port of the second circulation section 4012, the first circulation section 4011 is in conductive communication with the second circulation section 4012.

Taking a direction of placement of the connection module 4 as shown in Fig. 3 as a reference, the on-off assembly 402 in the present invention extends in a height direction of the connection module 4, and may slide upward in the height direction relative to the connection module 4 in the connection module 4. Therefore, in the initial position, the alignment hole of the on-off assembly 402 is misaligned with the port of the first circulation section 4011 and the port of the second circulation section 4012, and is located below the port of the first circulation section 4011 and the port of the second circulation section 4012.

When the port of the first circulation section 4011 needs to be in conductive communication with the port of the second circulation section 4012, a second pushing member of the detection module pushes the on-off assembly 402 upward, so that the alignment hole is in butt joint with the port of the first circulation section 4011 and the port of the second circulation section 4012, thereby achieving the conductive communication of the first flow channel 401, and enabling the solvent in the eluent chamber 107 to flow into the amplification tube 6.

When the solvent in the eluent chamber 107 completely flows into the amplification tube 6, the second pushing member pushes the on-off assembly 402 upward again, so that the alignment hole is misaligned with the port of the first circulation section 4011 and the port of the second circulation section 4012, thereby achieving the blocking of the first flow channel 401.

The on-off assembly 402 in the present invention may also be in the form of a baffle and a spring, that is, the baffle and the spring are both arranged in the connection module 4, and the baffle and the spring both extend in the height direction of the connection module 4, the alignment hole is formed in the baffle, and one end of the spring is connected to the baffle while the other end thereof abuts against an inner wall of the connection module 4.

When the first flow channel 401 needs to be in conductive communication, the second pushing member pushes the baffle upward, so that the alignment hole in the baffle may be aligned with the port of the first circulation section 4011 and the port of the second circulation section 4012, thereby achieving the conductive communication of the first flow channel 401, and the second pushing member always abuts against one end, away from the spring, of the baffle, so that the spring may always be in a compressed state and the first flow channel 401 remains in a conductive communication state.

When the first flow channel 401 needs to be blocked, the second pushing member is separated from the baffle, and the baffle returns to the initial state under the action of the spring, so that the alignment hole is misaligned with the port of the first circulation section 4011 and the port of the second circulation section 4012, thereby achieving the blocking of the first flow channel 401.

In this way, the structure of the on-off assembly 402 may be further optimized, that is, the on-off assembly 402 further includes two support plates respectively arranged on both sides of the baffle. One of the support plates supports the port of the first circulation section 4011, and the other support plate supports the port of the second circulation section 4012. The baffle is attached to the support plates and may move upward relative to the two support plates to achieve the alignment of the alignment hole.

By arranging the two support plates, on the one hand, the ends of the first circulation section 4011 and the second circulation section 4012 may be supported, so that the first circulation section 4011 and the second circulation section 4012 are more stable, and on the other hand, since the baffle is not in contact with the port of the first circulation section 4011 and the port of the second circulation section 4012, the problem that the first circulation section 4011 and the second circulation section 4012 are deformed due to the movement of the baffle, so that the alignment hole cannot be aligned to achieve conductive communication may be solved to a certain extent.

During the detection process, when the sealing film of the plurality of accommodating chamber is punctured by the magnetic rod sleeve assembly 3, the residual solvent in the plurality of accommodating chamber easily produces aerosol transmission, thereby polluting the detection environment in the detection module and affecting the detection result.

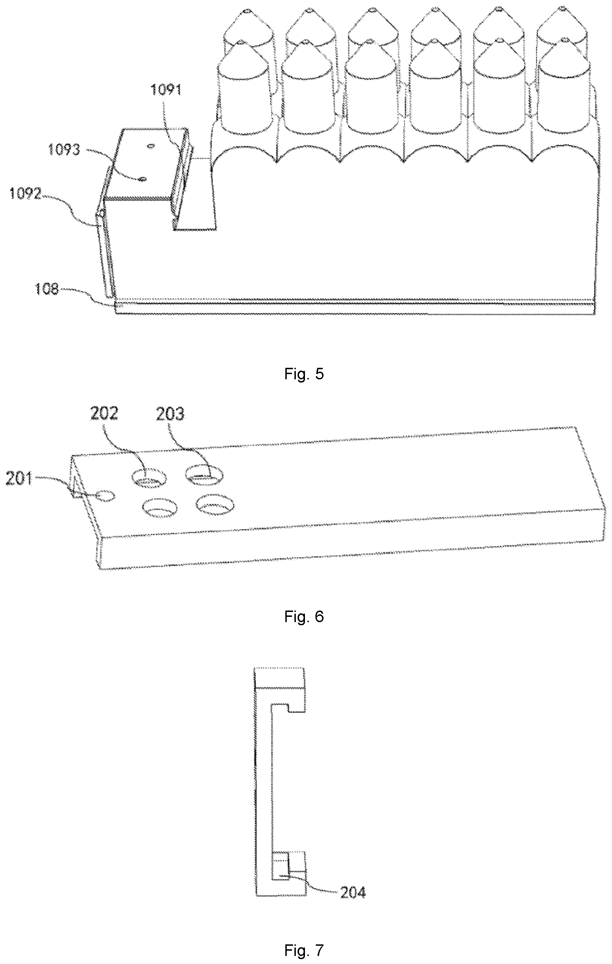

Therefore, in order to further avoid from affecting and polluting the detection environment due to the diffusion of the solvent in the plurality of accommodating chamber during the detection process, in an embodiment, as shown in Fig. 1 in combination with Figs. 6 -7, the reagent cartridge provided by the present invention further includes a slide cover 2. The slide cover 2 is slidably connected to the cartridge body 1 and covers the chamber openings of the plurality of accommodating chambers in the length direction of the cartridge body 1.

By arranging the slide cover 2, the detection module may control the slide cover 2 to move in the length direction of the cartridge body 1 during detection, so as to cover and seal the chamber openings of the plurality of accommodating chambers, thereby avoiding the problem that the detection environment is affected and polluted due to aerosol transmission of the residual solvent in the plurality of accommodating chambers to a certain extent, and improving the detection accuracy.

In an embodiment, an alignment part is formed on the slide cover 2 in the present invention, and the alignment part in the present invention is an alignment hole 201. When the reagent cartridge is not in use, the slide cover 2 is in an initial position relative to the cartridge body 1, a first perforation 202 is formed at a position of the slide cover 2 corresponding to the magnetic rod sleeve chamber 101 of the cartridge body 1, and a second perforation 203 is formed at a position corresponding to the sample chamber 102.

When in use, the first pushing member of the detection module may be inserted into the alignment hole 201 of the slide cover 2, so as to push the slide cover 2 to move relative to the cartridge body 1. Since the first perforation 202 and the second perforation 203 are formed in the slide cover in the present invention, and when the slide cover 2 is in the initial position, the first perforation 202 corresponds to the magnetic rod sleeve chamber 101, and the second perforation 203 corresponds to the sample chamber 102, in fact, a distance between the first perforation 202 and the second perforation 203 in the present invention is consistent with a distance between two adjacent accommodating chambers. After the sample is injected into the sample chamber 102, since the sealing film covering the sample chamber 102 is punctured, in order to avoid the sample in the sample chamber 102 from being polluted or aerosol transmission during the process of installing the reagent cartridge into the detection module and the process of inserting the magnetic rod sleeve assembly 3 into the magnetic rod and entering the magnetic bead preservation liquid chamber 103 to adsorb the magnetic beads, in actual operation, after the sample is injected, the second perforation 203 is blocked again by the sealing film, so that the pollution of the sample may be avoided to a certain extent.

In order to ensure that the slide cover 2 may always cover and seal the punctured sealing chamber during the process of cooperating with the movement of the magnetic rod sleeve assembly 3, in the present invention, a distance between the second perforation 203 in the slide cover 2 and one end, away from the second perforation 203, of the slide cover 2 is not less than a distance between the sample chamber 102 and one end, away from the sample chamber 102, of the cartridge body 1.

Before detection, the slide cover 2 correspondingly covers the cartridge body 1, the first perforation 202 corresponds to the magnetic rod sleeve 101, and the second perforation 203 corresponds to the sample chamber 102. When the detection starts, taking a viewing angle direction in Fig. 1 as an example, the detection module first removes the magnetic rod sleeve assembly 3 from the magnetic rod sleeve chamber 101, and controls the slide cover 2 to move left until the magnetic bead preservation liquid chamber 103 is exposed. The detection module controls the magnetic rod sleeve assembly 3 to enter the magnetic bead preservation liquid chamber 103 to absorb the magnetic beads. After the absorption is completed, the detection module controls the slide cover 2 to move left to expose the sample chamber 102, and controls the magnetic rod sleeve assembly 3 to enter the sample chamber 102 to absorb the sample.

After the absorption is completed, the detection module drives the magnetic rod sleeve assembly 3 to sequentially enter the first washing liquid chamber 104, the second washing liquid chamber 105, the standby chamber 106, and the eluent chamber 107, and finally returns to the magnetic rod sleeve chamber 101. The slide cover 2 moves synchronously, and sequentially covers the magnetic bead preservation liquid chamber 103, the first washing liquid chamber 104, the second washing liquid chamber 105, the standby chamber 106, and the eluent chamber 107. When the slide cover 2 covers the eluent chamber 107, the slide cover 2 returns to the initial position, that is, the first perforation 202 is aligned with the magnetic rod sleeve chamber 101, and the second perforation 203 is aligned with the sample chamber 102, so that the magnetic rod sleeve assembly 3 may enter the magnetic rod sleeve chamber 101.

In order to ensure that the slide cover 2 and the cartridge body 1 may only reciprocate in the length direction of the cartridge body 1, in the present invention, a first matching part 108 is formed on the cartridge body 1, and the first matching part 108 extends in the length direction of the cartridge body 1. A second matching part 204 is formed at a position of the slide cover 2 corresponding to the first matching part 108, and the first matching part 108 is matched with the second matching part 204, so that the slide cover 2 may slide relative to the cartridge body 1.

In an embodiment, as shown in Figs. 4 -7, in the present invention, the first matching part 108 on the cartridge body 1 is a first protrusion, and the second matching part 204 formed on the slide cover 2 is a first groove part. Through a clamped connection between the first protrusion and the first groove part, the slide cover 2 is limited in a width direction and a height direction of the cartridge body 1, so that the slide cover 2 may only move relative to the cartridge body 1 in the length direction of the cartridge body 1, thereby improving the stability of the overall device.

As shown in Figs. 1 -5 in combination with Fig. 11, in an embodiment, in the present invention, a matching protrusion 109 is formed at one end of the cartridge body 1, and a matching recess 405 is formed at a position of the connection module 4 corresponding to the matching protrusion 109. A third matching part 1091 and a fourth matching part 1092 are respectively formed on two side walls, in contact with the matching recess 405, of the matching protrusion 109, a fifth matching part 4051 matched with the third matching part 1091 is formed at a position of the matching recess 405 corresponding to the third matching part 1091, and a sixth matching part 4052 matched with the fourth matching part 1092 is formed at a position corresponding to the fourth matching part 1092.

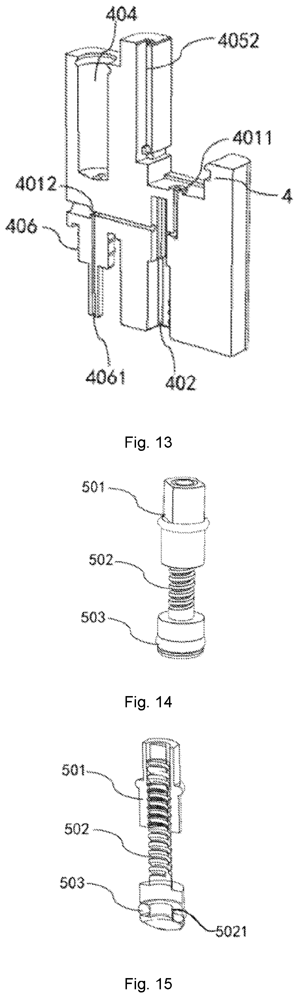

Through the matching protrusion 109 formed at the position of the cartridge body 1 corresponding to the connection module 4 and the matching recess 405 formed at the position of the connection module 4 corresponding to the matching protrusion 109 in the present invention, accurate butt joint of the cartridge body 1 and the connection module 4 may be achieved. In order to further improve the stability of the connection between the cartridge body 1 and the connection module 4, the third matching part 1091 and the fourth matching part 1092 are formed on the two side wall, in contact with the matching recess 40, of the matching protrusion 109 in the present invention, and as shown in Fig. 4 in combination with Fig. 5, the third matching part 1091 is a second protrusion extending in the width direction of the cartridge body 1, and the fourth matching part 1092 is a boss extending in the height direction of the cartridge body 1 and having channels formed on both sides. Correspondingly, as shown in Fig. 11 in combination with Fig. 13, the fifth matching part 4051 is a slot in clamped connection with the second protrusion, and the sixth matching part 4052 is a groove extending in the height direction of the connection module 4 and having third protrusions formed on positions on both sides corresponding to the channels.

The connection between the cartridge body 1 and the connection module 4 may be made more stable through the clamped connection between the second protrusion and the slot and the clamped connection between the boss and the groove.

It is to be noted here that the structural form provided above is only one of the implementations for achieving the stable connection between the cartridge body 1 and the connection module 4, clamping structures or inserting structures in other forms may also be configured to achieve the stable connection between the cartridge body 1 and the connection module 4, and the above manners are all within the scope of the conception of the present invention and will not be elaborated herein.

In an embodiment, as shown in Fig. 2 in combination with Fig. 3, in the present invention, a first sealing gasket 7 is arranged between the matching protrusion 109 and the matching recess 405, the eluent chamber 107 is formed in the matching protrusion 109, and one end of the first flow channel 401 passes through the matching recess 405. A conductive communication part is formed at a position of the first sealing gasket 7 corresponding to the communication part 1093, and one end of the conductive communication part communicates with the communication part 1093 while the other end thereof communicates with the first flow channel 401.

Since the cartridge body 1 and the connection module 4 are of split structures, and the eluent chamber 107 of the cartridge body 1 needs to be in conductive communication with the first flow channel 401 of the connection module 4, by arranging the first sealing gasket 7 between the matching protrusion 109 and the matching recess 405, and correspondingly forming the conductive communication part in the first sealing gasket 7, it may be ensured to a certain extent that the communication part 1093 of the eluent chamber 107 tightly communicates with one end of the first flow channel 401, thereby avoiding the problem of liquid seepage to a certain extent.

In an embodiment, as shown in Fig. 11 in combination with Fig. 12, in the present invention, a positioning part 406 is formed at a position of the connection module 4 corresponding to the amplification tube 6, and a tube opening of the amplification tube 6 is sleeved on the positioning part 406. A first flow guide tube 4061 and a second flow guide tube 4062 are formed on the positioning part 406, the first flow channel 401 is formed in the first flow guide tube 4061, and the second flow channel 403 is formed in the second flow guide tube 4062.

Through the positioning part 406 formed at the position of the connection module 4 corresponding to the amplification tube 6, the tube opening of the amplification tube 6 may be tightly sleeved on the positioning part 406, thereby improving the airtightness inside the amplification tube 6 after the amplification tube 6 is connected to the connection module 4.

In an embodiment, as shown in Fig. 2, in the present invention, a second sealing gasket 8 is further arranged between the amplification tube 6 and the connection module 4. Through the second sealing gasket 8, the sealing degree between the connection module 4 and the amplification tube 6 may be further improved.

The first flow guide tube 4061 and the second flow guide tube 4062 formed on the positioning part 406 may facilitate the formation of the first flow channel 401 and the second flow channel 403 in conductive communication with the amplification tube 6. Since the first flow guide tube 4061 and the second flow guide tube 4062 are formed, the problem of interference between the first flow channel 401 and the second flow channel 403 may be avoided to a certain extent.

It is to be noted here that, as shown in Fig. 2 in combination with Fig. 11, the first flow channel 401 and the second flow channel 403 in the connection module 4 in the present invention are both formed by injection molding, and in order to facilitate the injection molding of the first flow channel 401 and the second flow channel 403, forming holes 407 are further formed in positions of the connection module 4 corresponding to the first flow channel 401 and the second flow channel 403, and the forming holes 407 are filled with a blocking member 4071, so that the formed flow channel may be blocked to prevent leakage.

It is to be noted here that, in the above implementations, the plurality of accommodating chambers formed in the cartridge body 1 mainly have three sections, the first section is a rectangular section, the second section is a cylindrical section, and the third section is a conical section, but the accommodating chamber shown in the figures is only one of the implementations, and the depth, shape and size of the accommodating chamber are not specifically limited here and may be changed as required. The reagents correspondingly stored in the accommodating chambers are injected into the corresponding accommodating chambers and uniformly heat-sealed with an aluminum film, so as to avoid the problem that each reagent is polluted before detection to a certain extent.

It is to be further noted here that, as shown in Fig. 3, the depth of the eluent chamber 107 in the present invention is less than that of the other six accommodating chambers, and the communication part 1093 reserved at the bottom of the eluent chamber 107 has a communication hole with a diameter of 1 mm to 1.5 mm.

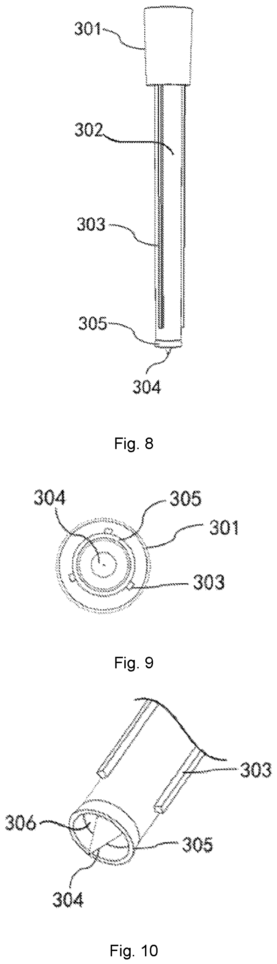

In an embodiment, as shown in Figs. 8 -10, the magnetic rod sleeve assembly 3 provided by the present invention includes a sleeve body 302, a fixed section 301, a side wing 303 and an anti-lost magnetic ring 305. The fixed section 301 is connected to one end of the sleeve body 302, a through hole is formed in the fixed section 301, an accommodating groove is formed in the sleeve body 302, the through hole communicates with the accommodating groove, and a puncture tip 30 is formed at the other end of the sleeve body 302. The side wing 303 extend in a length direction of the sleeve body and are arranged on an outer wall surface of the sleeve body 302. The anti-lost magnetic ring 305 is arranged at one end of the sleeve body 302 away from the fixed section 301 and forms an accommodating recess 306 with the puncture tip 304.

The fixed section 301 is configured to facilitate a moving mechanism of the detection module to grasp during detection. The fixed section 301 is actually of a sleeve structure with both ends penetrated, so as to communicate with a notch of the accommodating groove of the sleeve body 302, so that the magnetic rod may penetrate into the sleeve body 302.

In the present invention, one end of the sleeve body 302 away from the fixed section 301 is a sealed end, so as to avoid direct contact between the magnetic rod and the solvent in the accommodating chamber. In addition, the puncture tip 304 is further formed at the sealed end, so that the sealing film covering the cartridge body 1 may be punctured by the puncture tip 304.

In the present invention, a plurality of side wings 303 are further arranged on the outer wall surface of the sleeve body 302, and in an embodiment, as shown in Fig. 9, the number of the side wings 303 in the present invention is three, and the side wings are arranged at intervals in a circumferential direction of the sleeve body 302. The side wings 303 may stir the reagent in the accommodating chamber after the sleeve body 302 is inserted into the accommodating chamber, thereby increasing the mixing speed, increasing the stirring force, and making the mixing more uniform.

In the present invention, the anti-lost magnetic ring 305 arranged at one end of the sleeve body 302 away from the fixed section 301 may cooperate with the puncture tip 304 to form the accommodating recess 306, so that the captured magnetic beads may be located in the accommodating recess 306, thereby avoiding the problem that the magnetic beads are lost due to scratching of the sealing film during the transfer process to a certain extent.

In an embodiment, as shown in Fig. 14 in combination with Fig. 15, the movable member 5 provided by the present invention includes a fixed part 501, a movable member 502, and a ring sleeve part 503. The fixed part 501 is rotatably connected to an open end of the piston chamber 404, one end of the movable member 502 is rotatably connected to the fixed part 501, the ring sleeve part 503 is sleeved on the other end of the movable member 502, and an outer wall of the ring sleeve part 503 abuts against an inner wall of the piston chamber 404. When the fixed part 501 rotates relative to the piston chamber 404, the movable member 502 moves in an axial direction of the piston chamber 404 to drive the ring sleeve part 503 to move relative to the piston chamber 404.

In the present invention, the fixed part 501 is a threaded sleeve, and the threaded sleeve is rotatably connected to a port of the piston chamber 404, that is, the threaded sleeve may rotate relative to the piston chamber 404. The movable part 502 is a screw, one end of the screw is in threaded connection with the threaded sleeve, a bearing groove 5021 is formed at the other end of the screw, the bearing groove 5021 may accommodate the ring sleeve part 503, and the ring sleeve part 503 may be a rubber ring that abuts against the inner wall of the piston chamber 404 and has a certain elasticity.

When in use, a rotating assembly of the detection module may rotate the threaded sleeve. Since the threaded sleeve rotates relative to the piston chamber 404, the screw may reciprocate in a vertical direction, thereby driving the ring sleeve 503 to move in the piston chamber 404. Since the ring sleeve 503 abuts against the inner wall of the piston chamber 404, when the ring sleeve 503 moves upward, gas in the amplification tube 6 may be extracted through the second flow channel 403, so that a negative pressure is formed in the amplification tube 6, thereby achieving the purpose of the solvent in the eluent chamber 107 automatically entering the amplification tube 6 through the first flow channel 401.

It is to be noted here that the movable member 5 in the present invention is actually a piston rod that is able to move relative to the piston chamber 404. Therefore, in addition to the structure of the above movable member 5, other piston rod forms may also be configured, which will not be elaborated herein.

Some embodiments of the present invention further provide a detection device, including a detection module, a pipette, and the above reagent cartridge. The pipette injects a sample solvent to be detected into one of the plurality of accommodating chambers of the reagent cartridge, the reagent cartridge is connected to the detection module, and the sample solvent to be detected in the one of the plurality of accommodating chambers may be detected through the detection module.

When the detection device provided by the present invention is in use, the sample solvent to be detected is injected into a sample chamber 102 of the reagent cartridge through the pipette, and then the reagent cartridge carrying the sample solvent to be detected is installed on the detection module, so that the sample solvent to be detected may be detected through the detection module.

The detection module provided by the present invention includes a detection module body, a magnetic rod, a moving mechanism, a first pushing member, a second pushing member, a rotating assembly, and other structures. The detection module body may carry the reagent cartridge, the magnetic rod may be inserted into a magnetic rod sleeve assembly 3 of the reagent cartridge to adsorb magnetic beads carried in one of the plurality of accommodating chambers in a cartridge body 1 of the reagent cartridge, the moving mechanism may grab and move the magnetic rod sleeve assembly 3, the first pushing member may push a slide cover 2 of the reagent cartridge to slide relative to the cartridge body 1, and the second pushing member may push an on-off assembly 402 of the reagent cartridge in a connection module 4 of the reagent cartridge to move relative to the connection module 4 to achieve the on-off of a first flow channel 401 of the reagent cartridge, so that a sample may be detected quickly and safely through the structures contained in the above detection module.

The detection device provided by the present invention is a microfluidic nucleic acid detection device, and reagents used are nucleic acid detection reagents, including a set of nucleic acid extraction and purification reagents and a set of nucleic acid amplification detection reagents. The nucleic acid extraction and purification reagents include a lysis binding solution, a first washing liquid, a second washing liquid, magnetic beads, an eluent, a proteinase K, etc., and the nucleic acid amplification detection reagents are freeze-dried reagents containing reverse transcriptase, hot start Taq enzyme, dNTPs, primers, probes, etc.

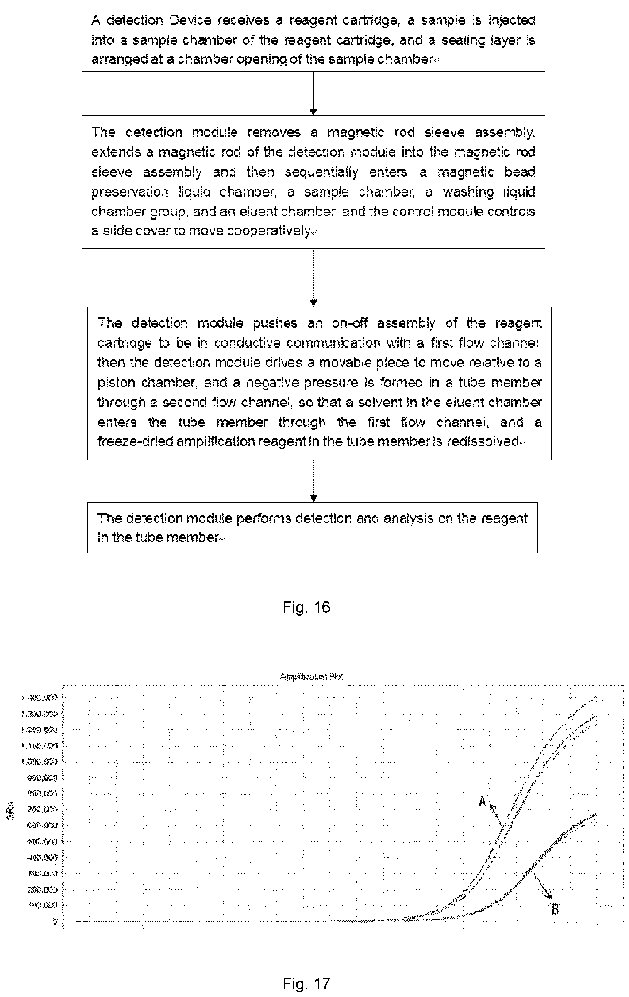

As shown in Fig. 16, the present invention further provides a detection method, including the following steps that: at S1, a sample is injected into a sample chamber 102 of a reagent cartridge, and a chamber opening of the sample chamber 102 is sealed with a sealing layer. In fact, when a slide cover 2 is arranged on the reagent cartridge, the chamber opening of the accommodating chamber of the reagent cartridge provided by the present invention is sealed with the sealing layer in the unused stage. When the sample needs to be injected into the sample chamber 102, the sealing layer is first punctured, then the sample is injected into the sample chamber 102, and then film sealing is performed on a second perforation 203 in the slide cover 2 to achieve the sealing of the sample chamber. Then, the reagent cartridge is arranged into a detection module. At S2, the detection module removes a magnetic rod sleeve assembly 3, extends a magnetic rod of the detection module into the magnetic rod sleeve assembly 3 and then sequentially enters a magnetic bead preservation liquid chamber 103, the sample chamber 102, a washing liquid chamber group, and an eluent chamber 107, and the detection module controls the slide cover 2 to move cooperatively. At S3, the detection module pushes an on-off assembly 402 to be in conductive communication with a first flow channel 401, and then the detection module drives a movable member 5 to move relative to a piston chamber 404, and forms a negative pressure in an amplification tube 6 through a second flow channel 403, so that a solvent in an eluent chamber 107 enters the amplification tube 6 through the first flow channel 401 to redissolve a freeze-dried amplification reagent in the amplification tube 6. At S4, the detection module performs detection and analysis on the reagent in the amplification tube 6.

S1 includes that: first, a sample to be detected and a negative control sample are sucked and respectively injected into two sample chambers 102 of the cartridge body 1, and film sealing is performed on a second perforation 203 of the slide cover 2.

300 µL of the sample to be detected, such as a nasopharyngeal swab preservation liquid sample or plasma, is sucked by a pipette, a sample solution to be detected is added to one of the sample chambers 102 of the cartridge body 1, and then 300 µL of a negative control sample solution is sucked and added to another sample chamber 102 of the cartridge body 1. The sample chamber 102 contains 500 µL of a lysis binding solution, and an upper layer of the lysis binding solution is covered with 100 µL of a liquid mineral oil component.

Second, the cartridge assembly with the sample to be detected and the negative control sample are connected to a detection module body of the detection module. After the cartridge body 1 is installed at the corresponding position of the detection module body, a heating module at the bottom of the sample chamber 102 starts heating at 65°C, and meanwhile, the detection module starts to perform S2.

S2 includes that: first, a moving mechanism of the detection module removes the magnetic rod sleeve assembly 3 from a magnetic rod sleeve chamber 101, the slide cover 2 moves left to expose the magnetic bead preservation liquid chamber 103, the magnetic rod of the detection module enters the magnetic rod sleeve assembly 3, and the moving mechanism moves the magnetic rod sleeve assembly 3 to the magnetic bead preservation liquid chamber 103 to adsorb magnetic beads.

The moving mechanism of the detection module is inserted into a fixed section 301 of the magnetic rod sleeve assembly 3, so that a sleeve body 302 may be moved out of the magnetic rod sleeve chamber 101 and transferred to above the magnetic bead preservation liquid chamber 103. The magnetic rod is submerged into the sleeve body 302 and then enters the bottom of the magnetic bead preservation liquid chamber 103 by puncturing a sealing film through a puncture tip 304, and the sleeve body 302 is rotated to adsorb the magnetic beads for 1 minute. The magnetic bead preservation liquid chamber 103 contains 100µL of a magnetic bead preservation liquid.

Second, the slide cover 2 moves left to expose the sample chamber 102, the moving mechanism transfers the magnetic rod sleeve assembly 3 into the sample chamber 102, lifts the magnetic rod to mix the adsorbed magnetic beads with the solvent in the sample chamber 102, and then extends the magnetic rod to adsorb the magnetic beads.

The magnetic rod sleeve assembly 3 with the magnetic beads is transferred to the sample chamber 102, the magnetic rod is lifted, the sample, the lysis binding solution, and the magnetic beads are mixed at a medium speed using the rotation of the sleeve body 302, and incubated for 6 minutes, then the magnetic rod is submerged into the sleeve body 302, a cyclic magnetic suction mode is selected for adsorption for 90 seconds, and then S5 is performed.

Third, the slide cover 2 moves right to cover the magnetic bead preservation liquid chamber 103, the moving mechanism transfers the magnetic rod sleeve assembly 3 from the sample chamber 102 into the first washing liquid chamber 104, lifts the magnetic rod to mix the magnetic beads with the solvent in the first washing liquid chamber 104, and then extends the magnetic rod to absorb the magnetic beads.

The magnetic rod sleeve assembly 3 is transferred to above the first washing liquid chamber 104 and enters the bottom of the first washing liquid chamber 104 by puncturing the sealing film through the puncture tip 304, the magnetic rod is lifted, the magnetic rod sleeve assembly 3 is rotated to quickly mix for 30 seconds, and the magnetic rod is submerged into the magnetic rod sleeve assembly 3 to adsorb the magnetic beads for 30 seconds. The first washing liquid chamber 104 contains 600 µL of a first washing liquid, and an upper layer of the first washing liquid is covered with 100 µL of the liquid mineral oil component.

Fourth, the slide cover 2 moves right again to cover the first washing liquid chamber 104, and the moving mechanism transfers the magnetic rod sleeve assembly 3 from the first washing liquid chamber 104 into the second washing liquid chamber 105, lifts the magnetic rod to mix the magnetic beads with the solvent in the second washing liquid chamber 105, and then extends the magnetic rod to absorb the magnetic beads.

The magnetic rod sleeve assembly 3 is transferred to above the second washing liquid chamber 105 and enters the bottom of the second washing liquid chamber 105 by puncturing the sealing film through the puncture tip 304, the magnetic rod is lifted, the magnetic rod sleeve assembly 3 is rotated to quickly mix for 30 seconds, and the magnetic rod is submerged into the magnetic rod sleeve assembly 3 to adsorb the magnetic beads in a circulation mode for 30 seconds. The second washing liquid chamber 105 contains 700 µL of a second washing liquid, and an upper layer of the second washing liquid is covered with 100 µL of the liquid mineral oil component.

Fifth, the slide cover 2 moves right again to cover the second washing liquid chamber 105, and the moving mechanism transfers the magnetic rod sleeve assembly 3 from the second washing liquid chamber 105 into a standby chamber 106, so that an end of the magnetic rod sleeve assembly 3 is in contact with the solvent in the standby chamber 106.

The magnetic rod sleeve assembly 3 is transferred to above the standby chamber 106 and enters the bottom of the standby chamber 106 by puncturing the sealing film through the puncture tip 304, the magnetic rod sleeve assembly 3 is rotated to mix quickly for 5 seconds, and the magnetic rod sleeve assembly 3 is quickly lifted. The standby chamber 106 contains 300 µL of a third washing liquid, and an upper layer of the third washing liquid is covered with 100 µL of the liquid mineral oil component.

Sixth, the slide cover 2 moves right to cover the standby chamber 106, the moving mechanism transfers the magnetic rod sleeve assembly 3 from the standby chamber 106 into the eluent chamber 107, so that the end of the magnetic rod sleeve assembly 3 is in contract with the solvent in the eluent chamber 107, lifts the magnetic rod to mix the magnetic beads with the solvent in the eluent chamber 107, and then extends the magnetic rod to adsorb the magnetic beads.

The magnetic rod sleeve assembly 3 is transferred to above the eluent chamber 107 and enters the bottom of the eluent chamber 107 by puncturing the sealing film through the puncture tip 304, the magnetic rod is lifted, the magnetic rod sleeve assembly 3 is rotated to slowly mix for 3 minutes, and the magnetic rod is submerged into the sleeve body 302 to adsorb the magnetic beads for 1 minute. The eluent chamber 107 contains 50 µL of an eluent, and an upper layer of the eluent is covered with 10 µL of the liquid mineral oil component.

At S3, the slide cover 2 moves right to cover the eluent chamber 107, so that the slide cover 2 is reset, the moving mechanism transfers the magnetic rod sleeve assembly 3 into the magnetic rod sleeve chamber 101, a second pushing member of the detection module pushes the on-off assembly 402 to be in conductive communication with a first circulation section 4011 and a second circulation section 4012, the movable member 5 moves relative to the piston chamber 404, and a negative pressure is formed in the amplification tube 6 through the second flow channel 403, so that the solvent in the eluent chamber 107 enters the amplification tube 6 through the first flow channel 401 to redissolve the freeze-dried amplification reagent in the amplification tube 6.

The magnetic rod sleeve assembly 3 is transferred to above the magnetic rod sleeve chamber 101, and at the same time, the first pushing member controls the slide cover 2 to move right to cover the eluent chamber 107, so that a first perforation 202 of the slide cover 2 may slide to the position corresponding to the magnetic rod sleeve chamber 101, the magnetic rod sleeve assembly 3 is withdrawn into the magnetic rod sleeve chamber 101, and then S4 is performed.

At S4, the detection module performs detection and analysis on the reagent in the amplification tube 6.

In S4, the second pushing member pushes the on-off assembly 402 to be in conductive communication with the first flow channel 401, and a rotating assembly rotates a threaded sleeve to drive a screw to move upward, so that a negative pressure is formed in the amplification tube 6, and the reagent in the eluent chamber 107 is completely transferred into the amplification tube 6 to redissolve the freeze-dried amplification reagent in the amplification tube 6.

Then, the detection module starts a temperature control module and an optical detection module to perform amplification program control on the amplification tube 6, an instrument starts to automatically perform PCR amplification detection, automatically collects a fluorescence signal in each circular reaction, presents the collected signal on a software interface in real time through optimization processing, completes fluorescence curve analysis after 30 minutes, automatically gives an analysis result, and prompts an operator to issue a detection report.

After the detection is completed, the detection module prompts to remove the reagent cartridge, which is then arranged in a ziplock bag and correspondingly treated as medical instrument waste. Then, the instrument automatically turns on a UV cleaning mode, and automatically turns off a power supply after 15 minutes or prompts the operator to start the next round of detection.

It is to be noted here that the detection reagent uses an influenza A virus-specific detection reagent. According to the above operating steps, the actions of nucleic acid extraction of 300 µL of a pseudovirus simulation sample and mixing of the amplification reagent are manually completed, and then 50 µL of the system is sucked from the mixed amplification reagent and arranged in ABI7500 for fluorescent PCR amplification. At the same time, the nucleic acid of 300 µL of the pseudovirus simulation sample is extracted by using a conventional virus nucleic acid extraction reagent and eluted by using 50 µL of the eluent, 10 µL of the extraction product is configured as a template and 50 µL of the amplification system is arranged in ABI7500 for amplification. The amplification result is configured as a control. The amplification result shows that the reagent cartridge, the detection device, and the detection method provided by the present invention may detect 3 to 4 cycles in advance. As shown in Fig. 17, in the figure, A is a result curve obtained by using the reagent cartridge and the detection device provided by the present invention according to the detection method, and B is a curve obtained by the traditional qPCR method. It may be seen from Fig. 17 that the present invention significantly improves the detection rate for low-abundance samples, and the detection sensitivity is much higher than that of the conventional detection method.

The above is only the optional embodiments of the present invention and is not intended to limit the present invention. Any modifications, equivalent replacements, improvements and the like made within the spirit and principle of the present invention shall fall within the scope of protection of the present invention.

### Industrial Applicability

The present invention provides a reagent cartridge, a detection device, and a detection method, which relate to the technical field of nucleic acid detection, so as to simplify the structure of the reagent cartridge to a certain extent and reduce costs. The reagent cartridge provided by the present invention includes a cartridge body assembly, a tube member, a first flow channel, and an on-off assembly. A plurality of accommodating chambers are formed in the cartridge body assembly, a sealing layer is arranged at a chamber opening of at least one of the plurality of accommodating chambers, the plurality of accommodating chambers include an eluent chamber, and a communication part is formed at the bottom of the eluent chamber. The tube member is connected to the cartridge body assembly. The first flow channel is in conductive communication from the communication part to the tube member. The on-off assembly is arranged on the first flow channel, when the on-off assembly is located at a first position, the first flow channel is in conductive communication, and when the on-off assembly is located at a second position, the first flow channel is closed.

In addition, it is to be understood that the reagent cartridge, the detection device, and the detection method of the present invention are reproducible and may be configured in a variety of industrial applications. In an embodiment, the reagent cartridge, the detection device, and the detection method of the present invention may be configured in the technical field of nucleic acid detection.

## Claims

1. A reagent cartridge, comprising a cartridge body assembly, wherein a plurality of accommodating chambers are formed in the cartridge body assembly, a sealing layer is arranged at a chamber opening of at least one of the plurality of accommodating chambers, the plurality of accommodating chambers comprise an eluent chamber, and a communication part is formed at the bottom of the eluent chamber;
a tube member, the tube member is connected to the cartridge body assembly;
a first flow channel, the first flow channel is in conductive communication from the communication part to the tube member; and
an on-off assembly, the on-off assembly is arranged on the first flow channel, when the on-off assembly is located at a first position, the first flow channel is in conductive communication, and when the on-off assembly is located at a second position, the first flow channel is closed.

2. The reagent cartridge as claimed in claim 1, wherein the cartridge body assembly comprises a cartridge body and a connection module, the plurality of accommodating chambers are formed in the cartridge body, and the connection module is connected to one end, close to the eluent chamber, of the cartridge body; and
the first flow channel is formed in the connection module, a second flow channel is further formed in the connection module, one end of the second flow channel communicates with the tube member, a piston chamber is formed in the connection module, one end, away from the tube member, of the second flow channel communicates with the piston chamber, and a movable member that is able to move relative to the piston chamber is arranged in the piston chamber.

3. The reagent cartridge as claimed in claim 1 or 2, wherein a diameter of the first flow channel is 1 mm to 2 mm.

4. The reagent cartridge as claimed in any one of claims 1 to 3, wherein the reagent cartridge further comprises a magnetic rod sleeve assembly, wherein the magnetic rod sleeve assembly is arranged in one of the plurality of accommodating chambers.

5. The reagent cartridge as claimed in any one of claims 2 to 4, wherein the plurality of accommodating chambers are a magnetic rod sleeve chamber, a sample chamber, a magnetic bead preservation liquid chamber, and a washing liquid chamber group, and the magnetic rod sleeve chamber, the sample chamber, the magnetic bead preservation liquid chamber, the washing liquid chamber group, and the eluent chamber are sequentially arranged from one end, away from the connection module, of the cartridge body to one end, close to the connection module, of the cartridge body.

6. The reagent cartridge as claimed in claim 5, wherein the washing liquid chamber group at least comprises a first washing liquid chamber, a second washing liquid chamber, and a standby chamber, and the first washing liquid chamber is arranged close to the magnetic bead preservation liquid chamber, and the second washing liquid chamber and the standby chamber are sequentially arranged in a direction away from the first washing liquid chamber.

7. The reagent cartridge as claimed in claim 6, wherein there are at least two parallel magnetic rod sleeve chambers, at least two parallel sample chambers, at least two parallel magnetic bead preservation liquid chambers, at least two rows of parallel washing liquid chamber groups, and at least two parallel eluent chambers, the at least two parallel magnetic rod sleeve chambers, the at least two parallel sample chambers, the at least two parallel magnetic bead preservation liquid chambers, the at least two rows of parallel washing liquid chamber groups, and the at least two parallel eluent chambers are formed in the cartridge body.

8. The reagent cartridge as claimed in any one of claims 2 to 7, wherein there are at least two first flow channels, at least two piston chambers, at least two second flow channels, and at least two tube members, the at least two first flow channels, the at least two piston chambers, and the at least two second flow channels are formed in the connection module, and the at least two tube members are connected to the connection module.

9. The reagent cartridge as claimed in any one of claims 2 to 8, wherein the on-off assembly is movably connected to the connection module;
the on-off assembly separates the first flow channel into a first circulation section and a second circulation section, and an alignment hole is formed in the on-off assembly; and
when the on-off assembly moves relative to the connection module to align the alignment hole with a port of the first circulation section and a port of the second circulation section, the first circulation section is in conductive communication with the second circulation section.

10. The reagent cartridge as claimed in any one of claims 2 to 9, wherein a positioning part is formed at a position of the connection module corresponding to the tube member, and a tube opening of the tube member is sleeved on the positioning part; and
a first flow guide tube and a second flow guide tube are formed on the positioning part, the first flow channel is formed in the first flow guide tube, and the second flow channel is formed in the second flow guide tube.

11. The reagent cartridge as claimed in any one of claims 5 to 10, wherein the reagent cartridge further comprises a slide cover, wherein the slide cover is slidably connected to the cartridge body, and the chamber openings of the plurality of accommodating chambers are covered by the slide cover in a length direction of the cartridge body.

12. The reagent cartridge as claimed in claim 11, wherein an alignment part, a first perforation, and a second perforation are formed on the slide cover, the alignment part, the first perforation, and the second perforation are sequentially arranged from one end of the slide cover to the other end in a length direction of the slide cover, and a distance from the second perforation to one end, away from the second perforation, of the slide cover is not less than a distance from the sample chamber to one end, away from the sample chamber, of the cartridge body.

13. The reagent cartridge as claimed in claim 11 or 12, wherein a first matching part is formed on the cartridge body, the first matching part extends in the length direction of the cartridge body, a second matching part is formed at a position of the slide cover corresponding to the first matching part, and the first matching part is matched with the second matching part, so that the slide cover may slide relative to the cartridge body.

14. The reagent cartridge as claimed in any one of claims 2 to 13, wherein a matching protrusion is formed at one end of the cartridge body, and a matching recess is formed at a position of the connection module corresponding to the matching protrusion; and
a third matching part and a fourth matching part are respectively formed on two side walls, in contact with the matching recess, of the matching protrusion, a fifth matching part matched with the third matching part is formed at a position of the matching recess corresponding to the third matching part, and a sixth matching part matched with the fourth matching part is formed at a position corresponding to the fourth matching part.

15. The reagent cartridge as claimed in claim 14, wherein the third matching part and the fourth matching part are both matching buckles, and the fifth matching part and the sixth matching part are both matching slots.

16. The reagent cartridge as claimed in claim 14 or 15, wherein a first sealing gasket is arranged between the matching protrusion and the matching recess, the eluent chamber is formed in the matching protrusion, and one end of the first flow channel passes through the matching recess; and
a conductive communication part is formed at a position of the first sealing gasket corresponding to the communication part, and one end of the conductive communication part communicates with the communication part while the other end thereof communicates with the first flow channel.

17. The reagent cartridge as claimed in any one of claims 4 to 16, wherein the magnetic rod sleeve assembly comprises a sleeve body, a fixed section, a side wing, and an anti-lost magnetic ring;
the fixed section is connected to one end of the sleeve body, a through hole is formed in the fixed section, an accommodating groove is formed in the sleeve body, the through hole communicates with the accommodating groove, and a puncture tip is formed at the other end of the sleeve body;
the side wing extend in a length direction of the sleeve body and is arranged on an outer wall surface of the sleeve body; and
the anti-lost magnetic ring is arranged at one end, away from the fixed section, of the sleeve body and forms an accommodating recess with the puncture tip.

18. The reagent cartridge as claimed in any one of claims 2 to 17, wherein the movable member comprises a fixed part, a movable part, and a ring sleeve part;
the fixed part is rotatably connected to an open end of the piston chamber, one end of the movable part is rotatably connected to the fixed part, the ring sleeve part is sleeved on the other end of the movable part, and an outer wall of the ring sleeve part abuts against an inner wall of the piston chamber; and
when the fixed part rotates relative to the piston chamber, the movable part moves in an axial direction of the piston chamber to drive the ring sleeve part to move relative to the piston chamber.

19. A detection device, comprising a detection module, a pipette, and the reagent cartridge as claimed in any one of claims 1 to 18;
wherein the pipette injects a sample solvent to be detected into one of the plurality of accommodating chambers of the reagent cartridge, the reagent cartridge is connected to the detection module, and the sample solvent to be detected in the one of plurality of accommodating chambers may be detected through the detection module.

20. The detection device as claimed in claim 19, wherein the detection module comprises a detection module body, a magnetic rod, a moving mechanism, a first pushing member, a second pushing member, and a rotating assembly, and the detection module body carries the reagent cartridge, the magnetic rod is inserted into a magnetic rod sleeve assembly of the reagent cartridge to adsorb magnetic beads carried in one of the plurality of accommodating chambers in a cartridge body of the reagent cartridge, the moving mechanism grabs and moves the magnetic rod sleeve assembly, the first pushing member pushes a slide cover of the reagent cartridge to slide relative to the cartridge body, and the second pushing member pushes an on-off assembly of the reagent cartridge in a connection module of the reagent cartridge to move relative to the connection module to achieve the on-off of a first flow channel of the reagent cartridge.

21. A detection method, comprising the following steps:
receiving, by a detection device, a reagent cartridge, injecting a sample into a sample chamber of the reagent cartridge, and arranging a sealing layer at a chamber opening of the sample chamber;
removing, by the detection device, a magnetic rod sleeve assembly from the reagent cartridge, extending a magnetic rod of the detection device into the magnetic rod sleeve assembly, and then sequentially entering a magnetic bead preservation liquid chamber, a sample chamber, a washing liquid chamber group, and an eluent chamber of the reagent cartridge;
pushing, by the detection device, an on-off assembly of the reagent cartridge to be in conductive communication with a first flow channel, and the first flow channel communicates with the eluent chamber and a tube member
driving, by the detection device, a piston assembly communicating with the tube member on the reagent cartridge to form a negative pressure in the tube member, so that a solvent in the eluent chamber enters the tube member through the first flow channel, and the solvent redissolves a freeze-dried amplification reagent in the tube member; and
performing, by the detection device, detection and analysis on the reagent in the tube member.

22. The detection method as claimed in claim 21, wherein the detection method further comprises: controlling, by the detection device, the movement of a slide cover slidably connected to a cartridge body of the reagent cartridge to sequentially seal the magnetic bead preservation liquid chamber, the sample chamber, the washing liquid chamber group, and the eluent chamber after the magnetic rod sleeve assembly leaves.
